# EUROPEAN PATENT SPECIFICATION

(11) **EP 3 052 173 B1**
(45) Date of publication and mention of the grant of the patent: **01.11.2023**
(21) Application number: 14849500.5
(22) Date of filing: 24.09.2014
(51) Int. Cl.: A61M 19/00, A61F 7/00, A61N 1/28, A61F 7/02

(54) **PORTABLE THERMOELECTRIC COOLING DEVICE FOR THERAPEUTIC CRANIOCERVICAL HYPOTHERMIA**
TRAGBARE THERMOELEKTRISCHE KÜHLVORRICHTUNG FÜR THERAPEUTISCHE KRANIOZERVIKALE HYPOTHERMIE
DISPOSITIF PORTABLE DE REFROIDISSEMENT THERMOÉLECTRIQUE POUR L'HYPOTHERMIE CRÂNIO-CERVICALE THÉRAPEUTIQUE

(30) Priority: 30.09.2013 US 201361884932 P
(43) Date of publication of application: 10.08.2016
(73) Proprietor: THE REGENTS OF THE UNIVERSITY OF CALIFORNIA, Oakland, CA 94607-5200 (US)
(72) Inventor: VERGARA, Julio L., Los Angeles, California 90095-1751 (US); SERRANO CARMONA, Raul, Los Angeles, California 90095-1751 (US); RESTREPO, Lucas, Los Angeles, California 90095-1769 (US)
(74) Representative: Richards, John
(86) International application number: PCT/US2014/057276
(87) International publication number: WO 2015/048170

(56) References cited:
- WO-A2-02/064069
- DE-U1-202006 020 386
- JP-A- 2006 230 761
- US-A- 4 860 748
- US-A- 5 603 728
- US-A- 5 603 728
- US-A1- 2002 120 317
- US-A1- 2002 156 509
- US-A1- 2002 161 419
- US-A1- 2003 097 845
- US-A1- 2004 158 303
- US-A1- 2008 097 560
- US-A1- 2008 097 560
- US-B2- 6 840 955

## Description

### BACKGROUND

Hypothermia is a promising neuroprotective therapy to improve the outcome of patients with neurological injuries, including cardiac arrest, neonatal asphyxia, stroke, head trauma and seizures. Unfortunately, most available cooling devices are inefficient or impractical for the implementation of brain hypothermia in emergency situations. In general, existing cooling devices are burdensome, non-portable, whole body units which do not target the brain specifically. Cooling is mostly attained after about two hours delay, and many devices interfere with other therapies being performed, such as airway support. Hence, there is a pressing need for portable and effective cooling devices that are predominantly targeted to brain hypothermia.

Several non-invasive (external) and invasive (internal) cooling strategies have been used to induce hypothermia. Invasive cooling methods require percutaneous venous access, which inherently carry a risk for complications such as hemorrhage, thrombosis, and infection (local and / or systemic); in general, these devices cannot be readily utilized outside hospital facilities. External cooling techniques have the important advantage that they do not need percutaneous procedures, and / or extensive training of medical personnel for their implementation. These techniques include ice packs, cooling tents, fluid pads, blankets, cold water circulating helmets, and liquid coolant spray in the nasal cavity. A significant limitation of existing external cooling devices is that most of them are massive units that induce cooling to the entire body instead of targeting the brain specifically. This implies that patients usually require sedation (in the hospital setting) in order to guarantee tolerance to therapy. While this type of hypothermia may be beneficial when several organs are affected in cases of multi-system failure, it may be unnecessary and even deleterious in diseases of the brain proper like stroke and status epilepticus. In contrast, locally-delivered hypothermia is generally regarded as a safe intervention, not causing significant reductions in the body core temperature.

An issue of great importance is the implementation of small (portable) cooling devices that can be applied by paramedic personnel in the field, in view of them being the first responders to neurological emergencies. Many existing devices are too cumbersome to be transported in ambulances, and/or require electric supplies that are not available in the field. Although some currently available cooling therapies are applied directly to the head and moderately portable, such as nasopharyngeal spray cooling, they have serious drawbacks; for example, this type of cooling interferes with breathing, a fundamental concern when managing patients with acute neurological injuries when active delivery of oxygen through masks and endotracheal tubes is routinely required. Also, nasopharyngeal spray cooling was designed for short treatment periods of less than 30 minutes. Another cranial device (Sovika from HVM Medical) induces sympathetically mediated vasoconstriction with significant systemic hypertension and bradycardia which may be a source of concern for certain types of acute neurological injury, such as brain hemorrhage.

Accordingly, a need exists for a cooling device for therapeutic craniocervical hypothermia which can be specifically directed at the cranial and neck area, while being portable and non-invasive. The technology described herein fulfills those needs, while overcoming the shortcomings of previous devices.

### BRIEF SUMMARY

The invention is disclosed in independent claim 1. Preferred embodiments are disclosed in the dependent claims. The technology of this disclosure is a portable device for the induction of therapeutic brain hypothermia. The technology generally comprises two electronically-controlled heat transfer component devices: (1) a cooling/heating head gear (helmet), and (2) a cooling/heating neck collar. The main active components of both the helmet and the collar are thermoelectric cooler units (TECs) whose operation is controlled by an electronic control module (ECM). Heat dissipation from the TECs is attained by circulating coolant, such as water or other coolant, through the devices in a closed loop configuration to keep the TECs operating efficiently. Coolant flow and heat dissipation are ultimately achieved by the use of a water circulator/heat exchanger unit.

WO 02/064069 discloses a heat/cold treatment and devices used in pain treatment especially of acute tension headache and migraine which is based on the effect of the alternate application from the exterior to arterial and venous vessels of heat and cold for different intervals (seconds or minutes). The treatment dissolves the congestion in the blood vessels, the painful region is supplied with blood and the respective nerve tracts, synapses and conduction fibers are influenced in a pain-relieving manner. To this end, the housing or bands are provided with cooling and heating units/elements and cold/heat is supplied by feeding power or cold or hot water or corresponding coolants (oils) while providing a humid milieu which may contain a medicament.

US2002/156509 discloses a portable thermal control module (TCM) for use in rapid heating or cooling the body. This thermal control module creates or displaces heat through the Peltier effect. A system and method of dynamically controlling the temperature of a body using multiple TCMs is also provided. This system and method involves a flexible thermal control suit (TCS) and a microprocessor. US5603728 discloses a cooling helmet with thermo-electric elements being cooled by a closed loop cooling liquid.

The portable thermoelectric cooling device for therapeutic craniocervical hypothermia described in this disclosure is particularly well-suited as an emergency response tool to rapidly attain (i.e., in less than 15 minutes) local brain hypothermia on individuals by effectively extracting heat from the scalp and neck (carotid arteries) while preventing skin damage (frostbite). The high heat exchange capacity, low-voltage operation, and continuous monitoring of scalp and neck skin temperatures of the patient, such as with medical grade sensors assures efficiency and safety.

Further aspects of the technology will be brought out in the following portions of the specification, wherein the detailed description is for the purpose of fully disclosing preferred embodiments of the technology without placing limitations thereon.

### BRIEF DESCRIPTION OF THE SEVERAL VIEWS

### OF THE DRAWING(S)

The technology described herein will be more fully understood by reference to the following drawings which are for illustrative purposes only:
FIG. 1 is a block diagram of a portable thermoelectric cooling device for therapeutic craniocervical hypothermia according to an embodiment of the technology described herein.
FIG. 2 is a cross-section view of a helmet according to an embodiment of the technology described herein, showing the interconnection of a thermally conductive mesh, water circulation channels, and an elastomeric coating.
FIG. 3 is a schematic of an array of thermoelectric coolers according to an embodiment of the technology described herein, shown arranged in a series of branches.
FIG. 4 is a block diagram of control electronics for therapeutic hypothermia according to an embodiment of the technology described herein.
FIG. 5 is a block diagram of a therapeutic hypothermia device utilizing flexible TEC modules according to an embodiment of the technology described herein.
FIG. 6 is a block diagram of interconnecting flexible TEC modules according to an embodiment of the technology described herein.
FIG. 7A through FIG. 7C are perspective views of flexible TEC modules according to an embodiment of the technology described herein, showing an internal coolant passageway layer and TEC layer.
FIG. 8A and FIG. 8B are temperature graphs according to an embodiment of the technology described herein, comparing temperature of the helmet at 4 seconds and after 155 seconds after activating the therapeutic hypothermia helmet.
FIG. 9A and FIG. 9B are temperature graphs according to an embodiment of the technology described herein, comparing temperature of the collar at 4 seconds and after 155 seconds after activating the therapeutic hypothermia collar.
FIG. 10A and FIG. 10B are temperature graphs of simulated brain temperatures found according to an embodiment of the technology described herein, comparing brain temperatures after 4 seconds and after 15 minutes in response to use of the combination of inventive helmet and collar.
FIG. 11A through 11M is a schematic of electronic control circuits for controlling TEC devices in a therapeutic hypothermia device according to an embodiment of the technology described herein.

### DETAILED DESCRIPTION

The therapeutic portable thermoelectric cooling device is designed for the specific treatment of the head and neck in patients with acute neurological emergencies. It is also particularly well-suited for use in domiciliary treatment for individuals with chronic migraines and status epilepticus, and as a replacement for current (less sophisticated) external hypothermia devices in hospital facilities.

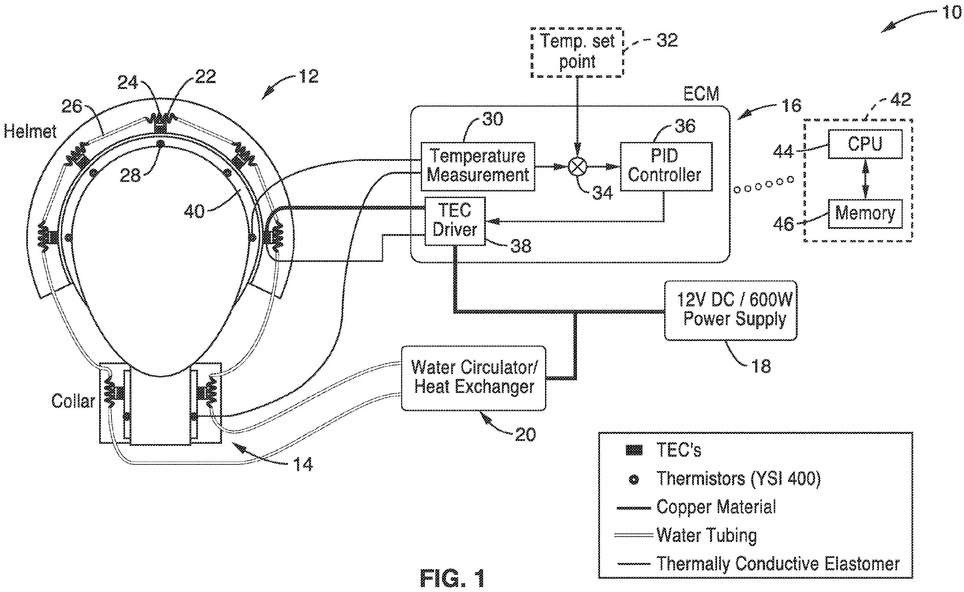

FIG. 1 illustrates an example embodiment 10 of the inventive portable thermoelectric cooling device. In this embodiment, a cooling and/or heating head gear (helmet) 12 is seen along with a cooling and/or heating neck collar (collar) 14. It should be appreciated that both the helmet and collar are configured as anatomically compliant structures. The helmet 12 and collar 14 are shown coupled to an electronic control module (ECM) 16, a power supply 18 and a combination coolant circulator and heat exchanger 20. In a preferred embodiment, power supply 18 is configured for making the device portable, as it is preferably compatible with AC/DC power available in ambulances, and/or to work as a stand-alone device powered with 12V batteries. Cooling and heating is preferably performed on the helmet and collar using a plurality of thermoelectric cooling devices (TECs) 22, with the extracted heat being collected by a thermally conductive collector 24 (e.g., copper structure) connected through fluid channels (e.g., tubing) 26 to the fluid circulator and heat exchanger 20. A series of temperature sensors 28 (e.g., medical thermistors [YSI 400 series compatible]) is also seen coupled near the interior surface of the helmet and collar for sensing the temperature closest to the skin of the patient.

It should be appreciated that the temperature sensors may be integrated into a removable thermal liner material, or alternatively, the temperature sensors may comprise individual disposable sensors that are directly coupled to the scalp/skin of the patient. A thermally conductive interior layer 40 is seen on the interior surface of the device, such as comprising a thermally conductive elastomer. It will be appreciated that these temperature sensors can be positioned in alignment with each TEC to sense the temperature of the patient skin at that location, or intermediate the TECs to sense skin temperature between TECs, or a combination of proximal the TECs with intermediary sensors between the TECs.

The electronic control module 16 is seen comprising a temperature measuring device 30 herein shown coupled to each of the temperature sensors 28 in the helmet and collar, such as using a series interface. A user interface 32 (i.e., human machine interface [HMI]) is preferably provided for setting temperature, such as a single temperature or a temperature profile with slightly different temperatures at different locations within the helmet and collar. In at least one embodiment, this user interface comprises a processing device and memory (e.g., microcontroller) along with a touch screen display (e.g., TFT-LCD touch screen). In at least one embodiment, device programming is configured to drive the TEC units based on a difference in temperature between temperature measured by the temperature sensors and a temperature set point, and to execute desired (programmed) patterns of cooling and heating cycles. Programming is configured for displaying temperature readings of the various probes, as well as deploying visual and auditory alarms and for managing device safety, such as for activating an automatic shutdown when conditions are detected which warrant it.

Temperature selection 32 operates through a combiner 34 acting as a subtractor in combination with temperature measurement 30, so that the amplitude of the difference signal between the temperature set point and the actual temperature drives the amplitude of the TEC response. Temperature control is performed through a controller 36, such as a proportional-integral-derivative (PID), connected to one or more thermoelectric cooler (TEC) drivers 38. The technology described herein allows the TECs to be driven in parallel or driven as separate rows of TECs, whereby one TEC driver is utilized for each group of TECs. A thermally conductive layer 40 is disposed for making contact with the scalp/skin of the patient. In at least one embodiment, this thermally conductive layer 40 may comprise a removable liner layer for the therapeutic unit, whether in a helmet, collar, or for conformal interface to other parts of the human body.

It should be appreciated that the electronic control module (ECM) 16 may alternatively incorporate at least one computer 42, such as comprising a central processing unit (CPU) 44 and memory 46, or a microcontroller or other means of executing programming for controlling TECs in response to measured temperatures and performing other desired system functions. The computer may be incorporated within the temperature set point control 32, or alternatively integrated into the body of the ECM 16.

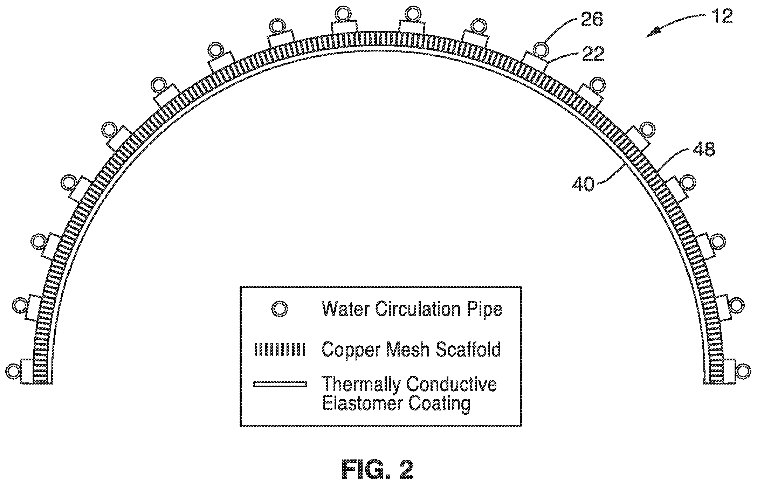

FIG. 2 depicts a cross section of an embodiment of the TEC helmet 12, showing a cross section on fluid passageways 26 (e.g., water circulation pipes), with at least one such passageway coupled to each of a plurality of thermoelectric coolers 22 which are coupled to a thermally conductive scaffolding structure 48 (e.g., copper mesh) which conducts heat received through a thermally conductive coating 40 (e.g., thermally conductive elastomeric coating) away from a patient (not shown).

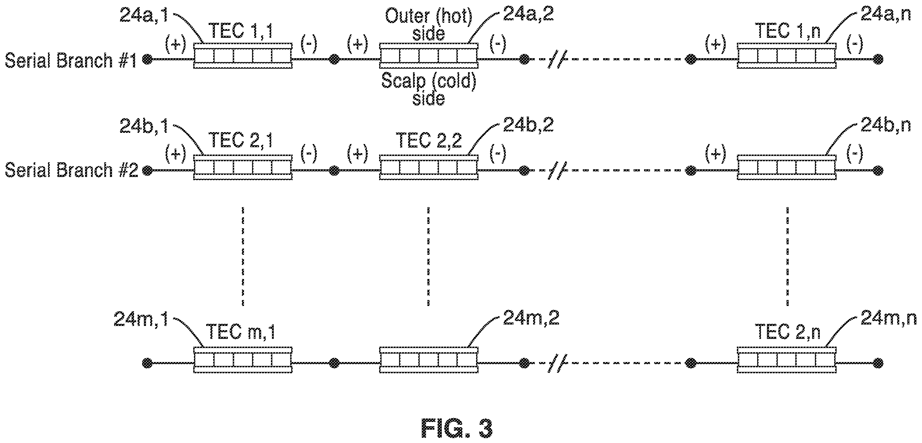

FIG. 3 illustrates an example embodiment of cooling/heating TECs 24ₐ₁, 24ₐ₂, 24ₐₙ, ...24_{b1}, 24_{b2}, 24_{bn}, and ...24ₘ₁, 24ₘ₂, 24ₘₙ, interconnected within the helmet organized with m banks of n devices per bank. The outer surface of the helmet (e.g., where the TEC heat dissipation sides are facing in this embodiment) were shown in FIG. 1 and FIG. 2 to be in contact with coolant fluid passageways coupled to a water circulator and heat exchanger. For simplicity of implementation, a water circulator (pump) and heat exchanger (e.g., external to helmet and collar) utilized for cooling a computer CPU can be utilized (e.g., Colace EX2-755). In using a simple heat exchanger of this type, the coolant is brought back to approximately room temperature and cycled back through to the passageways adjacent the TECs. The TEC helmet and collar are coupled to the heat exchanger, such as using flexible plastic tubing. Thus, when cooling a patient, each TEC unit cools its interior side toward the conductive inner layer adjacent the scalp/skin of the patient. The waste heat on the opposite side of each TEC is coupled into the cooling fluid and carried (i.e., pumped) to an external heat exchanger which extracts the heat and returns the coolant to absorb additional heat.

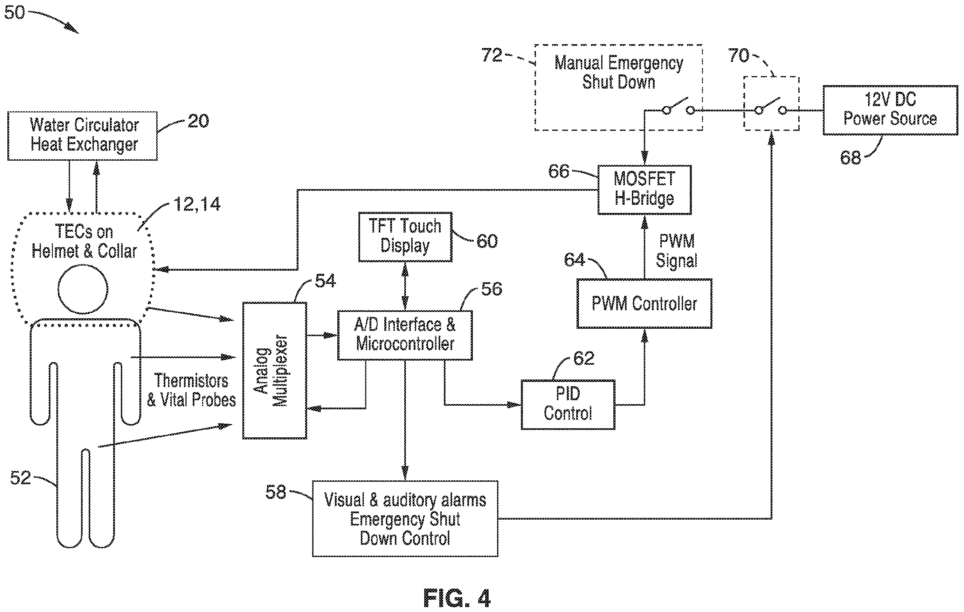

FIG. 4 illustrates an example embodiment 50 of control electronics based on a microcontroller. A patient 52 is seen which is coupled to the helmet 12 and collar 14, each having integrated TEC units thermally coupled to a water circulating heat exchanger 20. Additional sensors are coupled to the patient to monitor vital signs such as body temperature (thermistors), heart rate, blood pressure, and so forth. These sensors can be monitored by the inventive system through wired or wireless connection. Output from the sensors are registered (read) by the system, exemplified herein as being coupled through an analog multiplexer 54 coupled to a processor circuit 56 having an analog-to-digital (AD) converter and a microcontroller. It will be appreciated that some microcontrollers incorporate one or more AD and/or digital-to-analog (DA) converters. It will be further appreciated that a microcontroller includes both a central processing unit (CPU), memory and various input-output (IO) functionality, and is capable of executing programming for carrying out steps of an operational method. Alternatively, other forms of processors may be utilized without departing from the teachings of the technology described herein.

Coupled to the processor circuitry 56 is an alarm interface 58 which provides visual and auditory alarms as well as emergency shutdown control. A user interface 60 is coupled to the processor for controlling all desired aspects of operation and displaying information about the patient and system operation. In at least one embodiment, this user interface comprises a touch-based interface, such as a thin-film-transistor liquid crystal display TFT-LCD.

Processor circuit 56 also controls the amount of cooling or heating provided by the TECs in the helmet or collar devices. In FIG. 4 a proportional-integral-derivative (PID) circuit 62 is seen coupled to a pulse width modulating (PWM) controller 64, which outputs a PWM signal to a power switching circuit 66, herein exemplified as a MOSFET H-bridge coupled to the TEC elements in the helmet and collar. It should be appreciated that an H-bridge circuit allows outputting a desired positive or negative voltage/current, whereby the TECs can be operated to provide cooling, or alternatively heating, to the cranial and neck regions of the patient. It will be thus recognized that the technology described herein is not limited to cooling, although this is a more typical application. Circuit power is provided by at least one power source 68. Emergency module 58 is coupled to a switch (e.g., relay) 70 to allow power to be deactivated in response to conditions detected by processor circuit 56. In addition, a manual emergency shutdown switch 72 is shown allowing manual intervention to shut down the cooling devices.

It will be appreciated that a number of different circuit configurations may be utilized for controlling the operation of the inventive helmet and collar therapeutic cooling device. The TEC devices can be controlled in parallel, each receiving the same power from the H-bridge as shown in FIG. 1 and FIG. 4. Alternatively, the TEC elements may be configured in different areas of the helmet and collar, with each area driven by a separate PID control, PWM controller and H-bridge. Still further, modules having one or more TEC can be fabricated with each containing control circuitry for driving the TEC. In one example, each such module can be configured with an address, digital interface, and power control, such that processor 56 can individually control the cooling and heating of each TEC to assure that each area is cooled to the desired level within any desired temperature profile.

The following section describing an embodiment which utilizes flexible heat exchanging TEC modules.

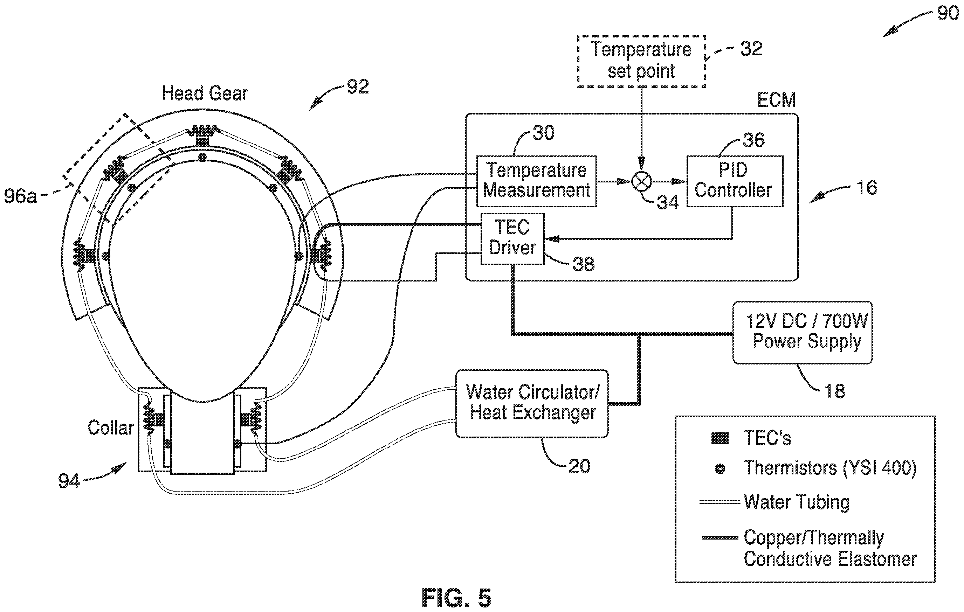

FIG. 5 illustrates an example embodiment 90 of the inventive helmet 92 and collar 94 utilizing flexible heat-exchanger modules (FHEMs) 96a that can be assembled and interconnected (i.e., series-connected or parallel-connected), such as by flexible tubing (e.g., plastic tubing, such as perfluoroalkoxy [PFA] heat conductive tubing) to build the helmet and collar. It will be appreciated that the same ECM 16, power supply 18 and heat exchanger 20 as described in FIG. 1 can be utilized. The ECM is again shown here with its temperature measurement module 30, a means for setting a temperature set point 32, a means 34 for using the difference between temperature set point and measured temperature to control a PID 36 whose output is directed through a TEC driver 38 coupled to TEC devices within each FHEM. Each FHEM is configured to have one or more TECs along with a thermistor, heat transfer passageways, and thermally conductive interior.

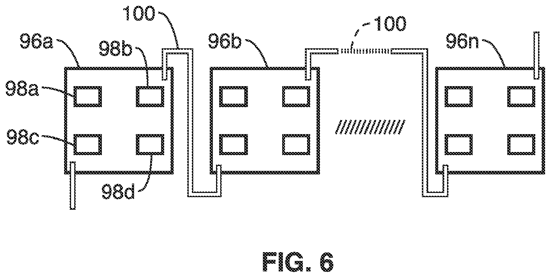

FIG. 6 illustrates interconnection of multiple modules 96a, 96b, and 96n, each having multiple TEC units, 98a, 98b, 98c, 98d interconnected by a fluid passageway 100 whose intake and outlet ends are coupled to a heat exchanger as seen in FIG. 5.

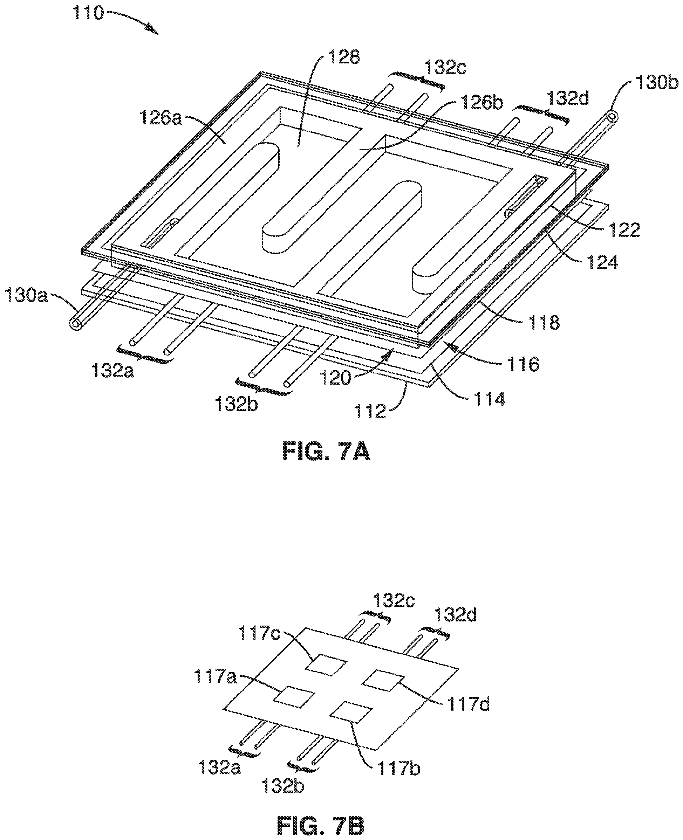

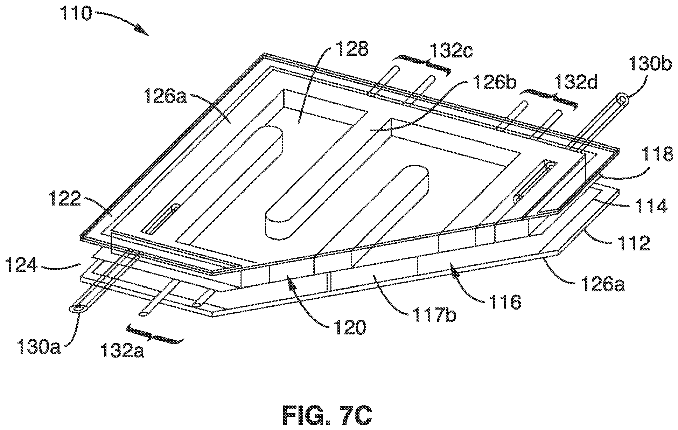

FIG. 7A through FIG. 7C illustrate an example embodiment 110 of a multi-layer structure of an FHEM, showing an upper layer with at least one fluid channel in FIG. 7A, a lower layer having multiple TEC units seen in FIG. 7B, and a cross-section in FIG. 7C, showing a better view of the relationship between the layers.

The layers generally comprise an elastomeric bottom layer 112 preferably comprising a soft heat conductive elastomer which interfaces skin of the patient with a conductive foil layer 114 (e.g., copper foil). A TEC device layer 116 is seen with one TEC 117b seen in the cross-section of FIG. 7C. In FIG. 7B the outline of four TECs 117a, 117b, 117c, and 117d can be seen in TEC device layer 116. Electrical connection pairs 132a, 132b, 132c and 132d, are seen in the figures for connecting each of the four TECs.

Another thermally conductive layer 118 is seen above TEC layer 116, which operates in concert with layer 122 to seal in a fluid flow layer 120. A flexible upper layer 124 provides strength, preferably comprising a reinforced elastomer, such as nylon-polydimethylsiloxane (PDMS) or other reinforced silicon sheet. An enclosed perimeter 126a of the fluid flow layer 120 is seen in FIG. 7A with protruding structures 126b, creating passageways 128 for extending the fluid path length for cooling fluid flow. Fluid is received into fluid layer 120 at a first fluid connection 130a, passes through a preferably serpentine chamber between perimeter 126a and protrusions 126b in fluid flow layer 120 and exits from second fluid connection 130b.

This flex-module aspect of the technology described herein can beneficially increase the flexibility, stretchability, and bendability of the active heat-transfer devices to assure that they closely follow the contours of the head (helmet) and neck (collar) and fit tightly against the scalp and neck skin toward assuring optimally efficient heat exchange.

It will be appreciated that typical heat exchange units (e.g., for cooling computer CPUs) are rigid, and made of solid metal blocks that are perforated to allow for rapid coolant flow. Instead, in this embodiment, each FHEM is soft and bendable, making them more conformable with human body contours. These features allow the inventive devices to be specifically built for the purposes of in-situ heating and cooling of not only the head and the neck, but also other human body components (e.g., legs, arms, chest, abdomen, or parts thereof) to which the inventive device is conformed. It should be appreciated that the cylindrical design shown for the collar can be sized and shaped to conform to these other body parts, in particular the legs, arms, chest, back, abdomen, and so forth.

### Working Helmet Prototype #0.

Prototypes have been constructed of the inventive therapeutic portable thermoelectric cooling device according to the embodiments described above. In a first prototype, a thermally conductive elastomer (e.g., SE4430 from Dow Corning^{®}) was used to coat a copper mesh scaffold with the shape of a human head to form the inner lining of the helmet (FIG. 2). The helmet utilized 50 TECs (e.g., Custom Thermoelectric^{®} 01711-5L31-06CF) having a size of approximately 15 mm x 15 mm each, which were symmetrically arranged on the outer surface of this scaffold. The TECs were electrically connected in 5 banks of 10 units in series as was seen in FIG. 3. The hot side of the TECs was thermally coupled to a network of miniature copper tubing that circulates water. A commercially available combination water circulator and heat exchanger was utilized (e.g., Koolance^{®} EX2-755; heat exchange power 500 - 600 watts). The electronic control module (ECM) was implemented with a full bridge controller (e.g., LTC1923 from Linear Technologies^{®}) for bi-directional current control using pulse-width modulation (PWM) and a proportional integrator-differentiator (PID) controller in the feedback loop. Each H-bridge was implemented using four MOSFETS (e.g., Si4564DY from Vishay Electronics^{®}). A 600 watt 12V DC power supply was utilized in this embodiment (e.g., PS-600 from Mean Well^{®}).

### Working Helmet Prototype #1.

This prototype utilized flexible heat-exchanging modules (FHEMs), as seen in FIG. 5 and FIG. 6, which were interconnected in a shape for the area being thermally treated (e.g., head, neck, and so forth). These assembled FHEM each accommodate and dissipate heat from 4 TEC units. The coolant (water) circulator was molded (at approximately 80 °C) using a thermally conductive elastomer (e.g., SE4430 or SE4430, Dow Corning) and custom-designed plastic molds in order to create channels between inlet and outlet tubing. The top of the circulator was made of polydimethylsiloxane (PDMS), such as Sylgard 184 or 182 from Dow Corning^{®}; or RTV-615 from Momentive Inc.^{®} reinforced with a nylon mesh; this cap was sealed onto the mold with thermally conductive adhesive (e.g., 3-1818, Dow corning). The floor of the circulator consisted of a copper foil (thickness range between approximately 0.004 - 0.015 inches (0.10 - 0.38 mm) ) adhered (e.g., glued directly such as with 3-1818 adhesive) onto the partitions of thermally conductive elastomer as seen in FIG. 7A through FIG. 7C.

The copper foil which constitutes the floor of the water circulator was directly adhered (e.g., glued) on top (hot side) of TECs (e.g., CustomThermoelectric 01711-5L31-06CF; 15 mm x 15 mm) symmetrically arranged. The bottom (cold) sides of the TECs were bonded (glued) directly onto another copper foil which was separated from the upper foil (bottom of the water chamber) by the body of the TEC and filled with heat insulation material such as neoprene, styrofoam, or insulating foam (not shown in the drawings of FIG. 7A through FIG. 7C). This arrangement provided effective heat isolation between the upper and lower copper foils while assuring flexibility and elasticity to the module. The bottom copper foil was covered with a thin layer (e.g., less than 0.03 inch (0.76 mm) thick) of soft heat conductive elastomer such as SE4445 CV Gel (Dow Corning) or SSP-1850C (Specialty Silicone Products Inc.^{®}). This layer constitutes a soft heat-transferring cushion to be in direct contact with the skin. Prototype #1 FHEM was found to have adequate structural flexibility for its adaption to the cranial surface while at the same time allowing for the necessary water circulation to dissipate sufficient heat.

### Simulation of Cooling Performance.

In order to theoretically analyze cooling performance of the combined helmet and neck collar devices, realistic mathematical 3D models were developed of a hypothetical head and neck, with geometry and dimensions typical of adults, using published parameters for metabolic heat generation, heat conductivity, blood flow, and so forth, for each anatomical organ.

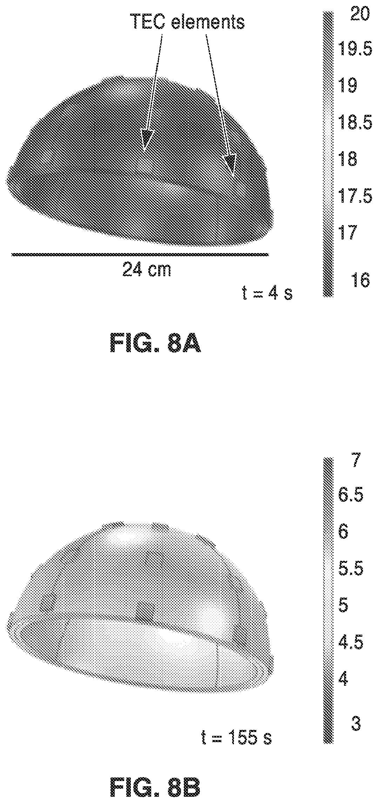

FIG. 8A and FIG. 8B depict a thermoelectric cooling helmet (e.g., this example being about 24 cm in diameter) in operation and showing TEC elements distributed over the area of the helmet. The appropriate heat diffusion-reaction equations for the helmet were solved while simulating an array of 25 TEC elements disposed on a conductive hemisphere cooling down the brain. An overall heat pumping power of approximately 125 watts for the 25 TECs; each TEC (0.25 inches square) (6.35 mm) with a maximal 6 watt heat pumping power. The model was solved using a finite element numerical solver (e.g., Comsol Mutiphysics^{®}). FIG. 8A shows that the helmet is still roughly at room temperature (about 20° C ) 4 seconds after it was turned on, but after only 155 s (about 2.5 minutes) of operation, it had cooled down to approximately 5° C.

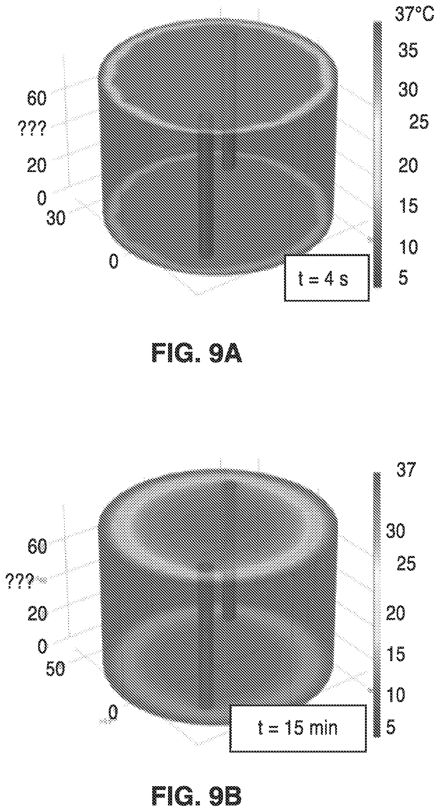

FIG. 9A and FIG. 9B depict thermoelectric cooling using the inventive neck collar. This 3D analysis shows the cooling effects after 4 seconds in FIG. 9A and after 15 minutes of collar operation in FIG. 9B. The calculated drop in temperature (input/output) of the blood flowing through the carotid artery is approximately 2° C (from 37° C to 35° C) when the skin is maintained at 5° C. The combined cooling effects of the helmet and neck collar on the brain temperature were calculated using the model.

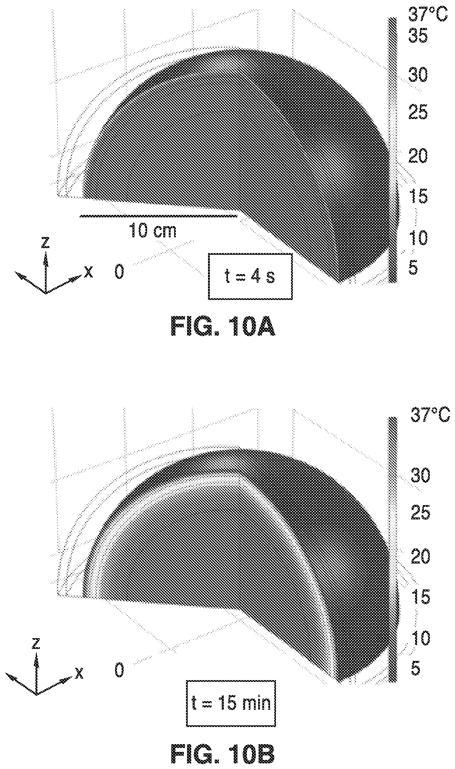

FIG. 10A and FIG. 10B show that the simultaneous cooling of the blood at the carotid artery using the inventive collar and of the brain using the inventive helmet leads to a rapid reduction in the temperature of the brain cortex to approximately 32° C within the first 15 minutes when the scalp is maintained to 5° C with the helmet.

### ECM Schematic Example.

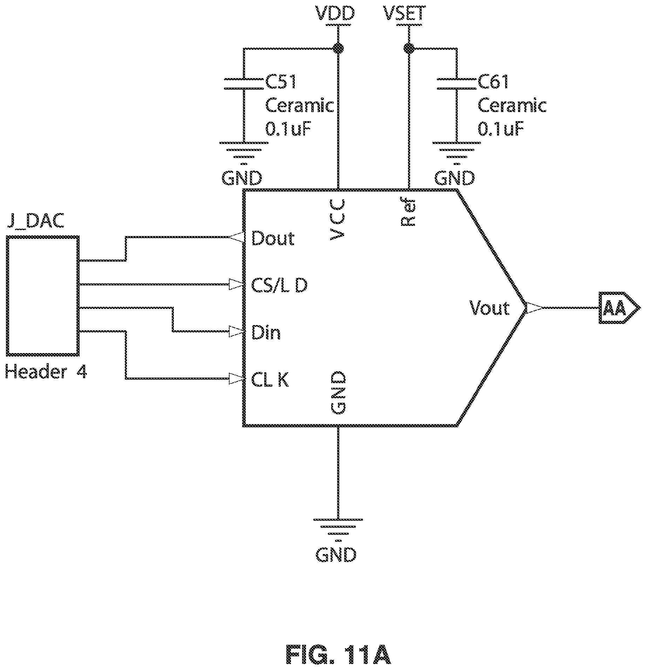

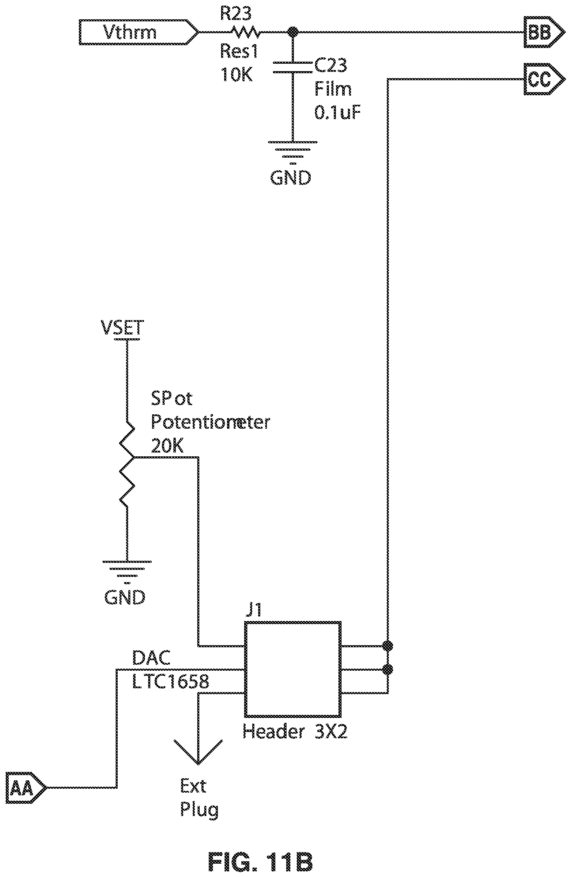

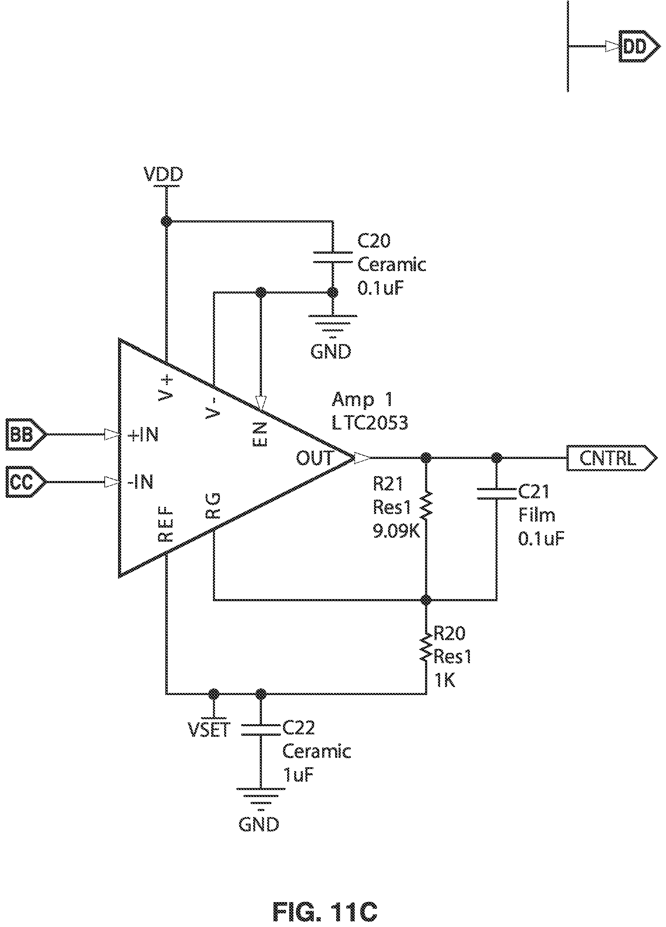

FIG. 11A through FIG. 11M illustrate an example embodiment of an ECM as previously described in FIG. 1, FIG. 4 and FIG. 5. In FIG. 11A are seen a digital-to-analog converter (DAC) (e.g., LTC1658 single supply, rail-to-rail voltage output, 14-bit digital-to-analog converter) having Data In (Dᵢₙ), clock (CLK), chip select/load (CS/LD), and data out (Dₒᵤₜ) in the digital side and outputting a voltage (Vₒᵤₜ) on the analog side. The DAC is seen connected to V_{DD}, having bypass capacitor C52, and to ground (Gnd). The DAC reference is seen connected to a reference signal (V_{SET}), which is also bypassed by a capacitor (C61) to ground. Input connections to the DAC are through J_DAC, with the output connected to header J1 seen in FIG. 11B along with a portion of the voltage from the reference voltage V_{SET} divided by a potentiometer (SPot), and in combination with resistor R23 and capacitor C23. Voltage from the DAC drives an amplifier circuit (Amp1) (e.g., LTC2053 Instrument amplifier), seen in FIG. 11C, in combination with resistors R20, R21, and capacitors C20, C21, and C22.

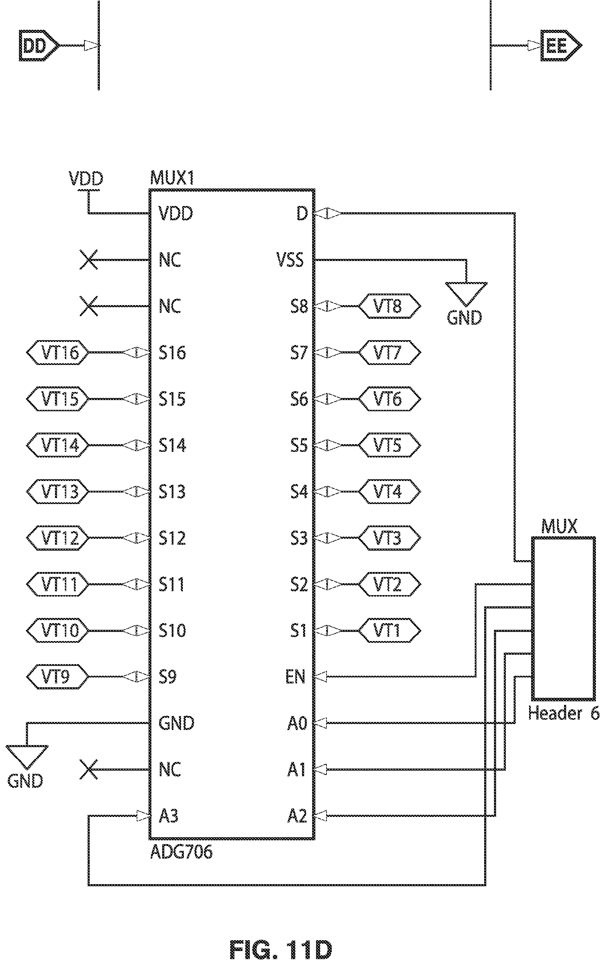

In FIG. 11D a header (MUX) is illustrated with an analog multiplexer (MUX1) (e.g., ADG706 16 input CMOS analog multiplexer) configured for selecting inputs from the temperature sensors of the thermoelectric cooling device.

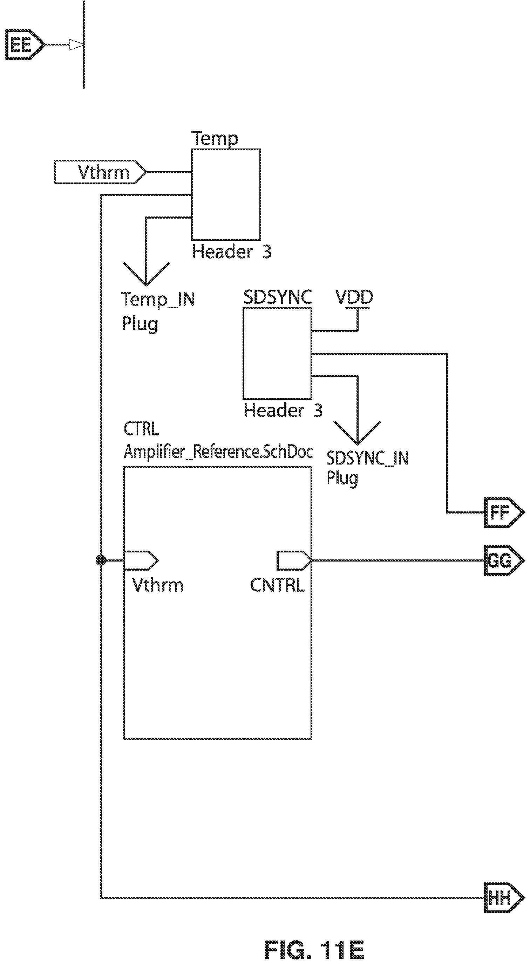

In FIG. 11E is illustrated the connection of the amplifier of FIG. 11C to header (Header 3) and other elements in receiving signal Vₜₕᵣₘ and outputting signal CNTRL.

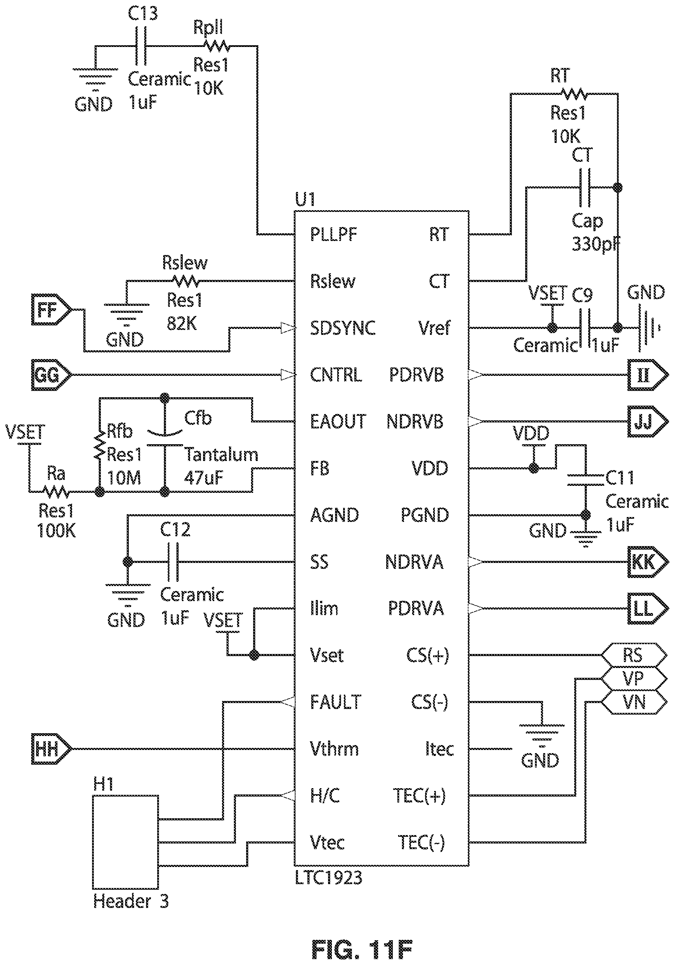

In FIG. 11F is shown a circuit for controlling thermoelectric coolers (U1) (e.g., LTC1923 pulse width modulator suited for thermoelectric cooler control) receiving the signal CNTRL from amplifier Amp1 as seen in FIG. 11E. The circuit of the bridge includes resistors Ra, Rpll, RT, and capacitors CT, C9, C11, C12, C13, Cfb, and is coupled to header H1.

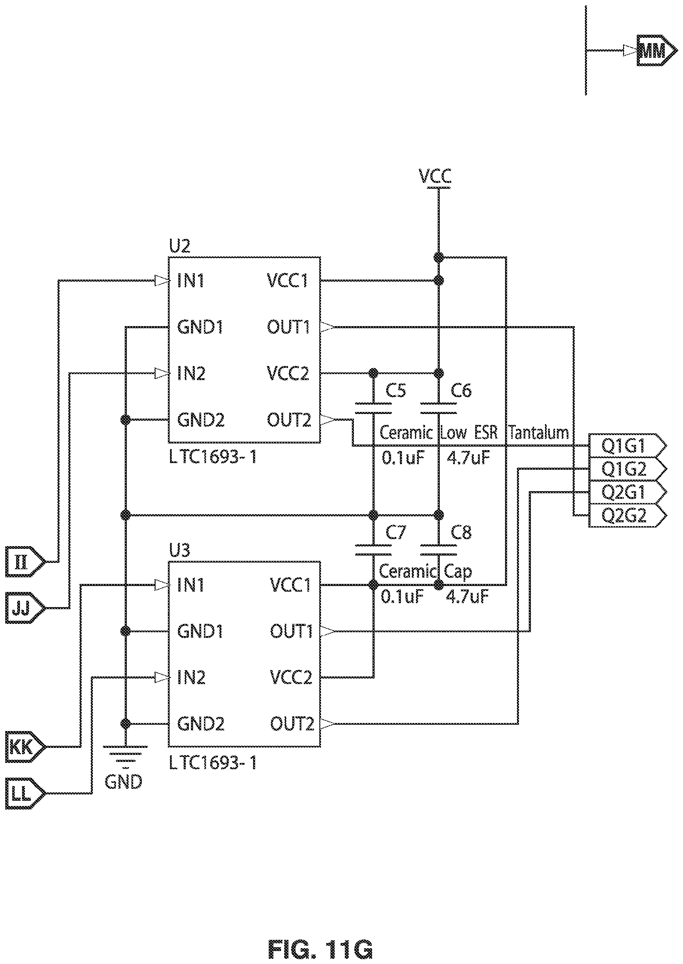

In FIG. 11G, the output from the TEC controller (bridge controller) in FIG. 11F is received and directed to N channel MOSFET drivers (U2 and U3), (e.g., LTC1693 dual N-channel MOSFET drivers) and capacitors C5, C6, C7 and C8.

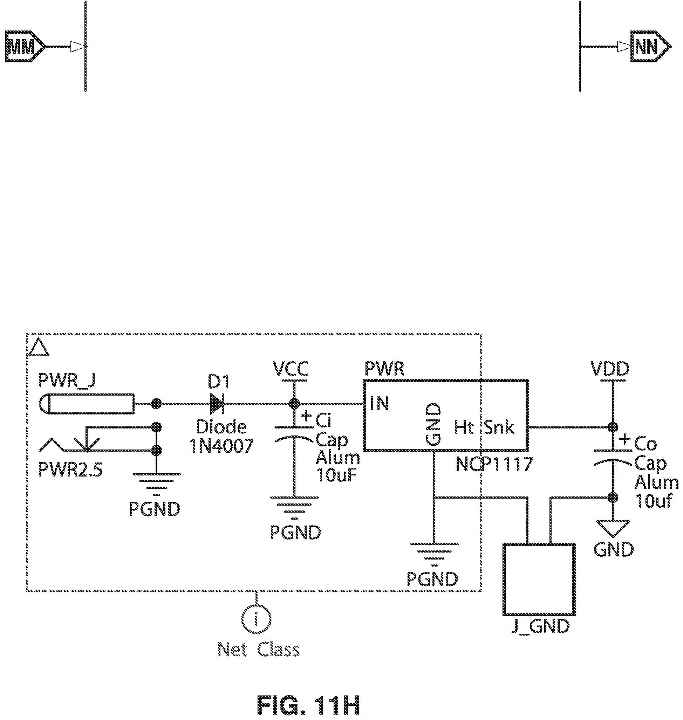

In FIG. 11H is seen a simple V_{DD} power supply circuit receiving power from a DC source, such as an automotive battery through a jack PWR_J, which passes through diode D1 to a storage capacitor (Ci) to voltage regulator PWR that outputs to another storage capacitor (Co) as a source of V_{DD}. Two different ground connections are seen being utilized from a header J_GND.

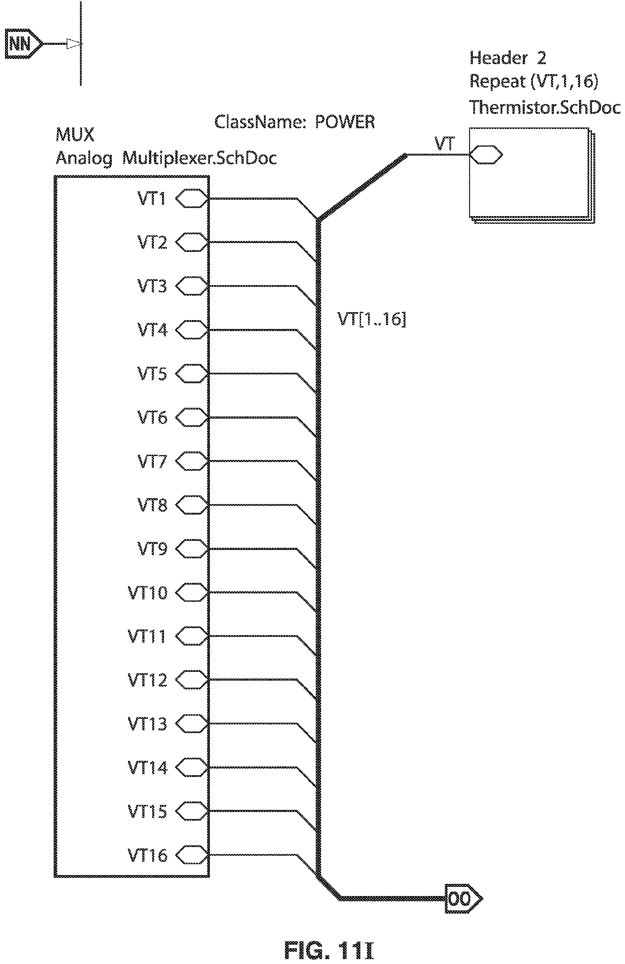

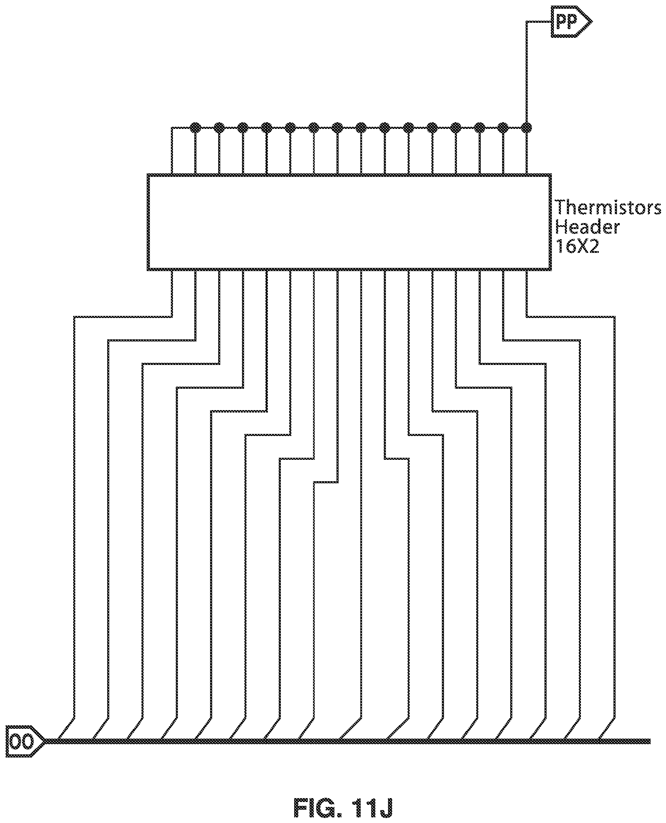

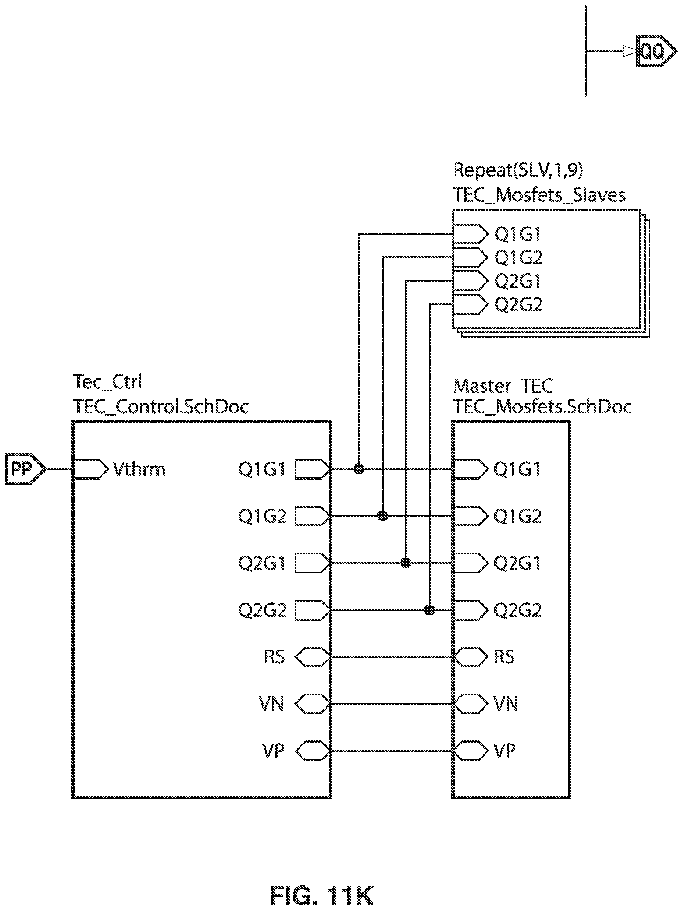

In FIG. 11I is seen a plurality of thermistors (Thermistor.SchDoc) configured for connection offsheet to MUX1 in FIG. 11D, and connected to a header (Thermistors) in FIG. 11J. Seen in FIG. 11K are connections RS, VP and VN from FIG. 11C, and the MOSFET drive signals Q1G1, Q1G2, Q2G1, Q2G2 from FIG. 11G.

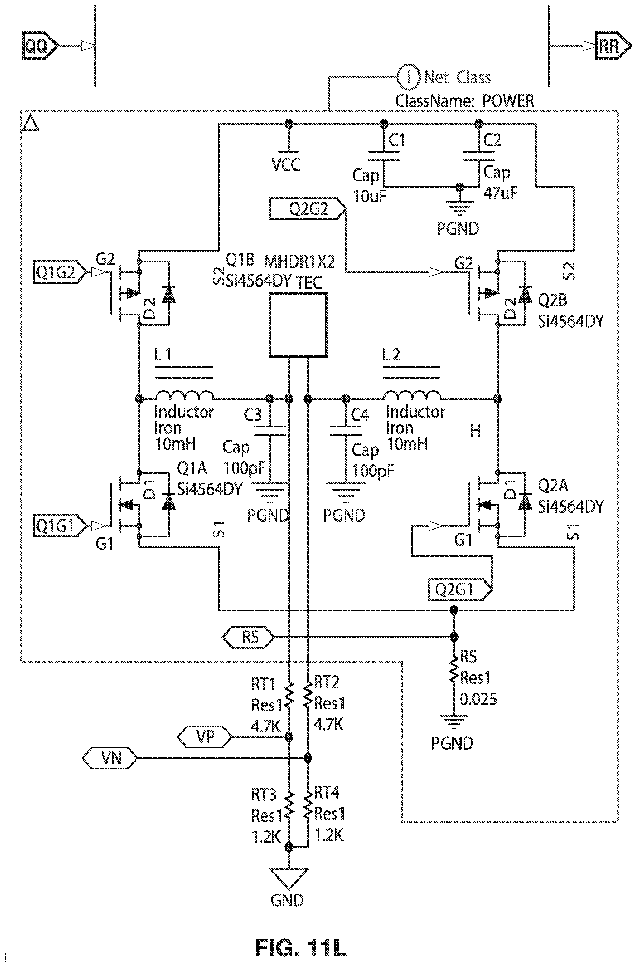

In FIG. 11L are depicted an example of a bi-directional MOSFET thermoelectric cooler drive circuit, seen receiving signal Q1G1, Q1G2, Q2G1, Q2G2. For the sake of simplicity of illustration, only a single MOSFET circuit is shown, although a plurality may be incorporated for driving groups of TECs, such as shown in FIG. 3. In the figure is seen an H-bridge of four MOSFETs Q1A, Q1B, Q2A, Q2B (e.g., Si4564DY MOSFETs), in a circuit utilizing inductors L1, L2, resistors RT1, RT2, RT3, RT4, RS, and capacitors C1, C2, C3, C4. Output from the H-bridge is configured for output to TECs through a header (TEC).

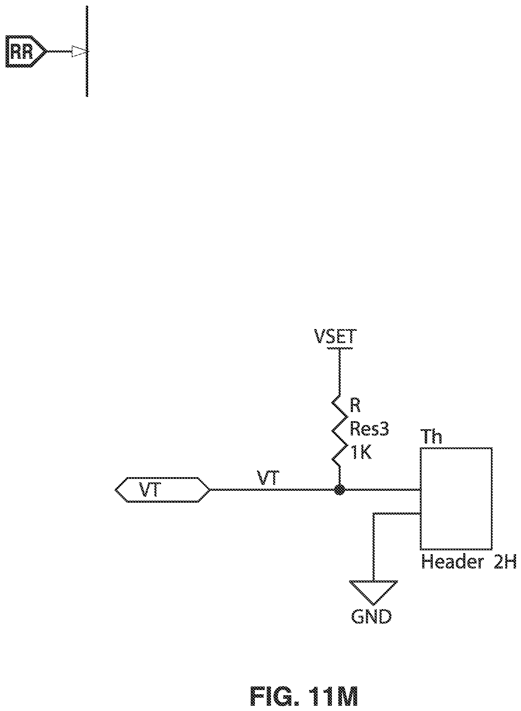

In FIG. 11M is seen a connection of a header to ground (GND) and a signal (VT) pulled up through resistor R to VSET.

It will be appreciated that the example ECM circuit described above can be implemented in a number of alternative ways without departing from the teachings of the technology described herein.

### Conclusion.

As will be appreciated from the foregoing discussion, currently available external cooling devices are not readily implemented, lack sufficient portability to be used in the field such as by paramedics, and have additional drawbacks. Furthermore, currently implemented cooling strategies have the significant limitation that induced hypothermia is too slow, resulting in a substantial time-gap for reaching meaningful brain cooling. This is a relevant issue because the neuroprotective effect of hypothermia is strongly influenced by the timing of therapy initiation. The gap between starting therapy and attaining brain hypothermia by about 3° C to 5° C (e.g., to 32° C to 34° C) takes, with current methodologies, longer than 30 minutes and in some cases as long as 2 hours. These delays can be explained in part by the difficulty in overcoming the heat contributed by blood circulation and the intrinsic heat production of the brain, but also (and importantly) by the fact that current devices are not specifically designed as heat extractors. Since these delays represent a lost opportunity to provide timely neuroprotection and local brain cooling, the technology described herein minimizes these delays with a craniocervical hypothermia device based on thermoelectric cooling (TEC) technology and an electronic temperature control servomechanism. Typically, cooling therapies have been implemented using open loop configurations in which temperature is manually monitored with thermometers, with nursing personnel changing the settings of slowly responding cooling devices. This approach is inefficient, tedious, and lends itself to the possibility of human error.

Accordingly, the technology described herein provides a portable solution to brain cooling comprising an electronically-controlled cooling (or heating) therapeutic units. These therapeutic units preferably comprise at least a helmet, and more preferably a helmet in combination with a neck collar. The helmet device is designed to extract heat from the brain in less than 15 minutes without causing local skin injury or the systemic discomfort and systemic complications of hypothermia. The neck collar device actively cools the neck in order to assist the baric cooling process by lowering the blood temperature at the carotid arteries by at least 1° C. The feasibility of the described embodiments are backed by extensive mathematical modeling that illustrate the advantages of the dual approach, while practical device implementations are described using state-of-the-art thermally conductive materials.

The thermoelectric medical cooling device described herein has several unique features. First, it is designed to induce local hypothermia in the brain and neck by taking advantage of the thermoelectric cooling effect. Unlike currently available massive cooling units requiring AC power to operate in order to cool down fluids circulating in helmets, the technology described herein is primarily conceived as a portable device, and is based on the use of active thermoelectric modules, ancillary electronic circuitry, and a low-voltage fluid circulator and heat exchanger, the combination being readily operated with standard 12V DC power. Furthermore, the method of using sophisticated feedback circuitry to fix (or clamp) the patient scalp temperature to values as low as 5° C (to avoid frostbite), while assuring that the hot side of the TECs are close to ambient temperatures (at about 20° C to 25° C), has been shown to be feasible with current technology. The inventive apparatus also permits warm to cold and vice versa transitions in a fast and safe manner, such as following programmed patterns. An important safeguard of the electronic control unit that automatically regulates the temperature of the cooling units, such as the helmet and collar, is that it allows flexible and accurate control of cooling and re-warming of the patients' brain by medical personnel.

Ultimately, while the helmet and neck collar devices are optimally designed to be ergonomic and capable of delivering brain hypothermia for patients with acute neurological emergencies who are seen in different clinical settings, they are particularly well-suited to be used on-site by paramedic personnel in ambulances, or by users who require portable heat exchange devices in various non-hospital settings (e.g., injured athletes at the sidelines sport venues, local hypothermia and/or hyperthermia at home, convalescent and nursing homes, and so forth).

Embodiments of the present technology may be described with reference to flowchart illustrations of methods and systems according to embodiments of the technology, and/or algorithms, formulae, or other computational depictions, which may also be implemented as computer program products. In this regard, each block or step of a flowchart, and combinations of blocks (and/or steps) in a flowchart, algorithm, formula, or computational depiction can be implemented by various means, such as hardware, firmware, and/or software including one or more computer program instructions embodied in computer-readable program code logic. As will be appreciated, any such computer program instructions may be loaded onto a computer, including without limitation a general purpose computer or special purpose computer, or other programmable processing apparatus to produce a machine, such that the computer program instructions which execute on the computer or other programmable processing apparatus create means for implementing the functions specified in the block(s) of the flowchart(s).

Accordingly, blocks of the flowcharts, algorithms, formulae, or computational depictions support combinations of means for performing the specified functions, combinations of steps for performing the specified functions, and computer program instructions, such as embodied in computer-readable program code logic means, for performing the specified functions. It will also be understood that each block of the flowchart illustrations, algorithms, formulae, or computational depictions and combinations thereof described herein, can be implemented by special purpose hardware-based computer systems which perform the specified functions or steps, or combinations of special purpose hardware and computer-readable program code logic means.

Furthermore, these computer program instructions, such as embodied in computer-readable program code logic, may also be stored in a computer-readable memory that can direct a computer or other programmable processing apparatus to function in a particular manner, such that the instructions stored in the computer-readable memory produce an article of manufacture including instruction means which implement the function specified in the block(s) of the flowchart(s). The computer program instructions may also be loaded onto a computer or other programmable processing apparatus to cause a series of operational steps to be performed on the computer or other programmable processing apparatus to produce a computer-implemented process such that the instructions which execute on the computer or other programmable processing apparatus provide steps for implementing the functions specified in the block(s) of the flowchart(s), algorithm(s), formula(e), or computational depiction(s).

It will further be appreciated that the terms "programming" or "program executable" as used herein refer to one or more instructions that can be executed by a processor to perform a function as described herein. The instructions can be embodied in software, in firmware, or in a combination of software and firmware. The instructions can be stored local to the device in non-transitory media, or can be stored remotely such as on a server, or all or a portion of the instructions can be stored locally and remotely. Instructions stored remotely can be downloaded (pushed) to the device by user initiation, or automatically based on one or more factors. It will further be appreciated that as used herein, that the terms processor, computer processor, central processing unit (CPU), and computer are used synonymously to denote a device capable of executing the instructions and communicating with input/output interfaces and/or peripheral devices.

Although the description herein contains many details, these should not be construed as limiting the scope of the disclosure but as merely providing illustrations of some of the presently preferred embodiments. Therefore, it will be appreciated that the scope of the disclosure fully encompasses other embodiments which may become obvious to those skilled in the art.

In the claims, reference to an element in the singular is not intended to mean "one and only one" unless explicitly so stated, but rather "one or more." All structural, chemical, and functional equivalents to the elements of the disclosed embodiments that are known to those of ordinary skill in the art are expressly incorporated herein by reference and are intended to be encompassed by the present claims. Furthermore, no element, component, or method step in the present disclosure is intended to be dedicated to the public regardless of whether the element, component, or method step is explicitly recited in the claims. No claim element herein is to be construed as a "means plus function" element unless the element is expressly recited using the phrase "means for". No claim element herein is to be construed as a "step plus function" element unless the element is expressly recited using the phrase "step for".

## Claims

1. An apparatus (10) for therapeutic hypothermia, comprising:
a plurality of thermoelectric cooling devices (22) distributed on a therapeutic unit (12, 90) configured as a helmet structure (12, 92) for attachment to one or more portions of a head and neck of a human body for creating a state of therapeutic craniocervical hypothermia to control cooling and re-warming;
at least one fluid passageway (26) disposed adjacent each of said thermoelectric cooling devices;
a water circulator and heat exchanger (20) configured for pumping a fluid through said at least one fluid passageway;
a thermally conductive layer (40) within said helmet structure configured to contact the scalp of a human body for thermal transfer between said thermoelectric cooling devices and the one or more portions of the head and neck of a human body;
a plurality of temperature sensors (28) coupled near the interior surface of said thermally conductive layer (40), said temperature sensors configured for sensing temperature of the skin; and
an electronic circuit (16) comprising a processor circuit configured for controlling said thermoelectric cooling devices (22) in response to a difference in temperature measured by each temperature sensor of said plurality of temperature sensors;
wherein said electronic circuit further comprises a full bridge controller for bi-directional current control using pulse-width modulation and a proportional integrator-differentiator controller in the feedback loop for controlling each of said plurality of thermoelectric cooling devices;
said plurality of thermoelectric cooling devices are grouped into more than one group, and
wherein each said group is controlled separately by said electronic circuit configured for controlling said thermoelectric cooling devices in response to a difference in temperature between temperature measured by said temperature sensors and a temperature set point, based on a temperature profile with slightly different temperatures at different locations, and for programmed patterns of cooling and heating cycles.

2. The apparatus of claim 1, wherein temperature is measured by each temperature sensor coupled to a processor circuit having an analog-to-digital converter as received through an analog multiplexer.

3. The apparatus of claim 1, wherein said apparatus is portable, and configured to operate with DC power sources, including 12V DC power sources.

4. The apparatus of claim 2, wherein said therapeutic unit (12, 90) comprises multiple flexible heat exchange modules (96a, 96b, 96n, 110), each flexible heat exchange module containing one or more thermoelectric cooling devices (22) and fluid passageways (26).

5. The apparatus of claim 1, wherein said thermally conductive layer (40) integrates said plurality of temperature sensors (28), and wherein said thermally conductive layer (40) comprises a removable liner layer which is removable from said therapeutic unit.

6. The apparatus of claim 4: wherein each said flexible heat exchange module of said helmet structure comprises flexible heat-exchanger modules (96a, 96b, 96n, 110) which are assembled and interconnected, either series-connected or parallel-connected, by flexible tubing to build said helmet structure; and
wherein each flexible heat exchange module is configured to have one or more TECs along with a thermistor, heat transfer passageways, and thermally conductive interior.

7. The apparatus of claim 1, further comprising a plurality of thermoelectric cooling devices distributed on a collar structure (14, 94), wherein said electronic circuit (16) drives said thermoelectric cooling devices in said helmet and said collar.

8. The apparatus of claim 6, wherein each said flexible heat exchange module (96a, 96b, 96n, 110) of said helmet structure comprises control circuitry for controlling the one or more TECs in said flexible heat exchange module.

9. The apparatus of claim 6, wherein said multiple flexible heat exchange modules (96a, 96b, 96n, 110) provide thermal transfer between thermoelectric cooling devices mounted to said flexible heat exchange module and said fluid; and
wherein each said flexible heat exchange module has internal fluid passageways (26, 100. 130a, 130b) between an inlet and an outlet configured for connection through an external fluid passageway to other flexible heat exchange modules and to said water circulator and heat exchanger.

10. The apparatus of claim 9, wherein said flexible heat exchange modules (96a, 96b, 96n, 110) are configured with at least two layers (110) comprising said thermoelectric cooling devices and said internal fluid passageways on different layers.

11. The apparatus of claim 1, further comprising a thermally conductive structure (48, 114) within said therapeutic unit, wherein said thermally conductive structure is disposed between said thermally conductive layer and said thermoelectric cooling devices.

12. The apparatus of claim 1, wherein said thermally conductive layer comprises a thermally conductive elastomeric material.

13. The apparatus of claim 11, wherein said thermally conductive structure (48, 114) comprises a mesh layer (48) to which said thermoelectric cooling devices (22) are attached.

14. The apparatus of claim 1, wherein said thermally conductive layer comprises a thermally conductive coating (40).

## Patentansprüche

1. Vorrichtung (10) zur therapeutischen Hypothermie, wobei die Vorrichtung folgendes umfasst:
eine Mehrzahl thermoelektrischer Kühlvorrichtungen (22), die auf einer therapeutischen Einheit (12, 90) verteilt sind, die als eine Helmstruktur (12, 92) zur Anbringung an einem oder mehreren Bereichen eines Kopfes und Halses eines menschlichen Körpers gestaltet ist, um einen Zustand der therapeutischen craniocervicalen Hypothermie zur Regelung der Kühlung und Wiedererwärmung zu erzeugen;
mindestens einen Fluidkanal (26), der angrenzend an jede der thermoelektrischen Kühlvorrichtungen angeordnet ist;
einen Wasserzirkulator und Wärmetauscher (20), der so gestaltet ist, dass er ein Fluid durch den mindestens einen Fluidkanal pumpt;
eine wärmeleitfähige Schicht (40) in der Helmstruktur, die so gestaltet ist, dass sie die Kopfhaut eines menschlichen Körpers für eine Wärmeübertragung zwischen den thermoelektrischen Kühlvorrichtungen und dem einen oder mehreren Bereichen des Kopfes und Halses eines menschlichen Körpers berührt;
eine Mehrzahl von Temperaturfühlern (28), die nahe der Innenseite der wärmeleitfähigen Schicht (40) gekoppelt sind, wobei die Temperaturfühler so gestaltet sind, dass sie die Temperatur der Haut messen; und
einen elektronischen Schaltkreis (16), der einen Prozessorschaltkreis umfasst, der gestaltet ist zur Steuerung der thermoelektrischen Kühlvorrichtungen (22) als Reaktion auf eine Differenz der Temperatur, die von jedem Temperaturfühler der Mehrzahl von Temperaturfühlern gemessen wird;
wobei der elektronische Schaltkreis ferner eine Vollbrückensteuereinheit zur bidirektionalen Stromsteuerung unter Verwendung von Impulsbreitenmodulation umfasst sowie eine proportionale Integrator-Differentiator-Steuereinheit in der Rückkopplungsschleife zur Steuerung jeder der Mehrzahl thermoelektrischer Kühlvorrichtungen;
wobei die Mehrzahl thermoelektrischer Kühlvorrichtungen in mehr als eine Gruppe gruppiert sind, und
wobei jede Gruppe separat durch den elektronischen Schaltkreis gesteuert wird, gestaltet zur Steuerung der thermoelektrischen Kühlvorrichtungen als Reaktion auf einen Temperaturunterschied zwischen der von den Temperaturfühlern gemessenen Temperatur und einem Temperatureinstellpunkt, auf der Basis eines Temperaturprofils mit leichter unterschiedlichen Temperaturen an verschiedenen Stellen, und für programmierte Muster von Kühl- und Heizzyklen.

2. Vorrichtung nach Anspruch 1, wobei die Temperatur durch jeden Temperaturfühler gemessen wird, der mit einem Prozessorschaltkreis gekoppelt ist, der einen Analog-Digital-Umsetzer aufweist, gemäß einem Empfang durch einen analogen Multiplexer.

3. Vorrichtung nach Anspruch 1, wobei die Vorrichtung portabel und für einen Betrieb mit Gleichstromenergiequellen gestaltet ist, einschließlich 12-Volt-Gleichstromenergiequellen.

4. Vorrichtung nach Anspruch 2, wobei die therapeutische Einheit (12, 90) mehrere flexible Wärmetauschermodule (96a, 96b, 96n, 10) umfasst, wobei jedes flexible Wärmetauschermodul eine oder mehrere thermoelektrische Kühlvorrichtungen (22) und Fluidkanäle (26) enthält.

5. Vorrichtung nach Anspruch 1, wobei die wärmeleitfähige Schicht (40) die Mehrzahl von Temperaturfühlern (28) integriert, und wobei die wärmeleitfähige Schicht (40) eine entfernbare Schutzschicht umfasst, die von der therapeutischen Einheit entfernt werden kann.

6. Vorrichtung nach Anspruch 4, wobei jedes flexible Wärmetauschermodul der Helmstruktur flexible Wärmetauschermodule (96a, 96b, 96n, 110) umfasst, die montiert und miteinander verbunden sind, entweder über Reihenschaltung oder Parallelschaltung durch flexible Schläuche, um die Helmstruktur zu bilden; und
wobei jedes flexible Wärmetauschermodul so gestaltet ist, dass es eine oder mehrere TECs in Verbindung mit einem Thermistor, Wärmeübertragungskanälen und einem wärmeleitfähigen Inneren aufweist.

7. Vorrichtung nach Anspruch 1, wobei diese ferner eine Mehrzahl thermoelektrischer Kühlvorrichtungen umfasst, die an einer Manschettenstruktur (14, 94) verteilt sind, wobei der elektronische Schaltkreis (16) die thermoelektrischen Kühlvorrichtungen in dem Helm und der Manschette steuert.

8. Vorrichtung nach Anspruch 6, wobei jedes flexible Wärmetauschermodul (96a, 96b, 96n, 110) der Helmstruktur eine Steuerschaltkreisanordnung zur Steuerung einer oder mehrerer TECs in dem flexiblen Wärmetauschermodul umfasst.

9. Vorrichtung nach Anspruch 6, wobei die mehreren flexiblen Wärmetauschermodule (96a, 96b, 96n, 110) für eine Wärmeübertragung zwischen den an dem flexiblen Wärmetauschermodul angebrachten thermoelektrischen Kühlvorrichtungen und dem Fluid sorgen; und
wobei jedes der flexiblen Wärmetauschermodule innere Fluidkanäle (26, 100, 130a, 130b) zwischen einem Einlass und einem Auslass aufweist, gestaltet für eine Verbindung über einen externen Fluidkanal mit anderen flexiblen Wärmetauschermodulen und mit dem Wasserzirkulator und Wärmetauscher.

10. Vorrichtung nach Anspruch 9, wobei die flexiblen Wärmetauschermodule (96a, 96b, 96n, 110) mit mindestens zwei Schichten (110) gestaltet sind, welche die thermoelektrischen Kühlvorrichtungen und die inneren Fluidkanäle auf verschiedenen Schichten umfassen.

11. Vorrichtung nach Anspruch 1, wobei diese ferner eine wärmeleitfähige Struktur (48, 114) in der therapeutischen Einheit umfasst, wobei sich die wärmeleitfähige Struktur zwischen der wärmeleitfähigen Schicht und den thermoelektrischen Kühlvorrichtungen befindet.

12. Vorrichtung nach Anspruch 1, wobei die wärmeleitfähige Schicht ein wärmeleitfähiges elastomeres Material umfasst.

13. Vorrichtung nach Anspruch 1, wobei die wärmeleitfähige Struktur (48, 114) eine Netzschicht (48) umfasst, an welcher die thermoelektrischen Vorrichtungen (22) angebracht sind.

14. Vorrichtung nach Anspruch 1, wobei die wärmeleitfähige Schicht einen wärmeleitfähigen Überzug (40) umfasst.

## Revendications

1. Appareil (10) pour hypothermie thérapeutique, comprenant :
une pluralité de dispositifs thermoélectriques de refroidissement (22) répartis sur une unité thérapeutique (12, 90) conçue comme une structure de casque (12, 92) à fixer sur une ou plusieurs parties de la tête et du cou d'un corps humain pour créer un état d'hypothermie crânio-cervicale thérapeutique afin de réguler le refroidissement et le réchauffement ;
au moins un passage de fluide (26) disposé à côté de chacun desdits dispositifs thermoélectriques de refroidissement ;
un circulateur d'eau et échangeur de chaleur (20) conçu pour pomper un fluide à travers ledit au moins un passage de fluide ;
une couche thermoconductrice (40) à l'intérieur de ladite structure de casque conçue pour entrer en contact avec le cuir chevelu d'un corps humain pour le transfert thermique entre lesdits dispositifs thermoélectriques de refroidissement et une ou plusieurs parties de la tête et du cou d'un corps humain ;
une pluralité de capteurs de température (28) couplés près de la surface intérieure de ladite couche thermoconductrice (40), lesdits capteurs de température étant conçus pour détecter la température de la peau ; et
un circuit électronique (16) comprenant un circuit de traitement conçu pour commander lesdits dispositifs thermoélectriques de refroidissement (22) en réponse à une différence de température mesurée par chaque capteur de température de ladite pluralité de capteurs de température ;
ledit circuit électronique comprenant en outre un dispositif de commande à pont complet pour la commande bidirectionnelle du courant à l'aide d'une modulation de largeur d'impulsion et un dispositif de commande intégrateur-différentiateur proportionnel dans la boucle de rétroaction pour commander chacun de ladite pluralité de dispositifs thermoélectriques de refroidissement ;
ladite pluralité de dispositifs thermoélectriques de refroidissement étant regroupés en plus d'un groupe, et
chaque groupe étant commandé séparément par ledit circuit électronique conçu pour commander lesdits dispositifs thermoélectriques de refroidissement en réponse à une différence de température entre la température mesurée par lesdits capteurs de température et un point de consigne de température, sur la base d'un profil de température avec des températures légèrement différentes à différents endroits, et pour des schémas programmés de cycles de refroidissement et de chauffage.

2. Appareil selon la revendication 1, la température étant mesurée par chaque capteur de température couplé à un circuit de traitement doté d'un convertisseur analogique-numérique et reçu par l'intermédiaire d'un multiplexeur analogique.

3. Appareil selon la revendication 1, ledit appareil étant portable et conçu pour fonctionner avec des sources d'alimentation en courant continu, y compris des sources d'alimentation de 12 V CC.

4. Appareil selon la revendication 2, ladite unité thérapeutique (12, 90) comprenant plusieurs modules d'échange de chaleur flexibles (96a, 96b, 96n, 110), chaque module d'échange de chaleur flexible contenant un ou plusieurs dispositifs thermoélectriques de refroidissement (22) et des passages de fluides (26).

5. Appareil selon la revendication 1, ladite couche thermoconductrice (40) intégrant ladite pluralité de capteurs de température (28), et ladite couche thermoconductrice (40) comprenant une couche de revêtement amovible qui peut être retirée de ladite unité thérapeutique.

6. Appareil selon la revendication 4 ; chacun desdits modules d'échange de chaleur flexibles de ladite structure de casque comprenant des modules d'échange de chaleur flexibles (96a, 96b, 96n, 110) qui sont assemblés et interconnectés, soit en série, soit en parallèle, par des tubes flexibles pour construire ladite structure de casque ; et
chacun desdits modules d'échange de chaleur flexibles étant conçu pour avoir un ou plusieurs dispositifs thermoélectriques de refroidissement avec un thermistor, des passages de transfert de chaleur et un intérieur thermiquement conducteur.

7. Appareil selon la revendication 1, comprenant en outre une pluralité de dispositifs thermoélectriques de refroidissement répartis sur une structure de collier (14, 94), ledit circuit électronique (16) commandant lesdits dispositifs thermoélectriques de refroidissement dans ledit casque et ledit collier.

8. Appareil selon la revendication 6, chacun desdits modules d'échange de chaleur flexibles (96a, 96b, 96n, 110) de ladite structure de casque comprenant un circuit de commande pour commander un ou plusieurs dispositifs thermoélectriques de refroidissement dans ledit module d'échange de chaleur flexible.

9. Appareil selon la revendication 6, lesdits plusieurs modules d'échange de chaleur flexibles (96a, 96b, 96n, 110) assurant le transfert thermique entre les dispositifs thermoélectriques de refroidissement montés sur ledit module d'échange de chaleur flexible et ledit fluide ; et
chacun desdits modules d'échange de chaleur flexibles ayant des passages de fluide internes (26, 100, 130a, 130b) entre une entrée et une sortie conçus pour une liaison à travers un passage de fluide externe à d'autres modules flexibles d'échange de chaleur et audit circulateur d'eau et échangeur de chaleur.

10. Appareil selon la revendication 9, lesdits modules d'échange de chaleur flexibles (96a, 96b, 96n, 110) étant conçus avec au moins deux couches (110) comprenant lesdits dispositifs thermoélectriques de refroidissement et lesdits passages de fluides internes sur différentes couches.

11. Appareil selon la revendication 1, comprenant en outre une structure thermoconductrice (48, 114) à l'intérieur de ladite unité thérapeutique, ladite structure thermoconductrice étant disposée entre ladite couche thermoconductrice et lesdits dispositifs thermoélectriques de refroidissement.

12. Appareil selon la revendication 1, ladite couche thermoconductrice comprenant un matériau élastomère thermoconducteur.

13. Appareil selon la revendication 11, ladite structure thermoconductrice (48, 114) comprenant une couche de mailles (48) à laquelle sont fixés lesdits dispositifs thermoélectriques de refroidissement (22).

14. Appareil selon la revendication 1, ladite couche thermoconductrice comprenant un revêtement thermoconducteur (40).
